# EUROPÄISCHE PATENTSCHRIFT

(11) **EP 4 305 016 B1**
(45) Veröffentlichungstag und Bekanntmachung des Hinweises auf die Patenterteilung: **30.04.2025**
(21) Anmeldenummer: 22711009.5
(22) Anmeldetag: 04.03.2022
(51) Int. Cl.: C07C 51/02, C07C 51/43, C07C 63/15, C07C 63/20, C07C 63/28, C07C 61/09, C07C 63/26, C07C 63/16, C07C 51/09

(54) **VERFAHREN ZUR GEWINNUNG AROMATISCHER DICARBONSÄUREN AUS IHREN METALLSALZEN**
PROCESS FOR EXTRACTING AROMATIC DICARBOXYLIC ACIDS FROM THEIR METAL SALTS
PROCÉDÉ D'EXTRACTION D'ACIDES DICARBOXYLIQUES AROMATIQUES À PARTIR DE LEURS SELS MÉTALLIQUES

(30) Priorität: 11.03.2021 DE 102021105970
(43) Veröffentlichungstag der Anmeldung: 17.01.2024
(73) Patentinhaber: RITTEC Umwelttechnik GmbH, 21339 Lüneburg (DE)
(72) Erfinder: MÜLLER, Clemens, 38104 Braunschweig (DE); BREPOHL, Esther, 38116 Braunschweig (DE); BIERMANN, Lars, 31177 Harsum (DE); EICHERT, Carsten, 21391 Reppenstedt-Dachtmissen (DE); SCHOLL, Stephan, 38116 Braunschweig (DE)
(74) Vertreter: Michalski Hüttermann & Partner Patentanwälte mbB
(86) Internationale Anmeldenummer: PCT/EP2022/055608
(87) Internationale Veröffentlichungsnummer: WO 2022/189302

(56) Entgegenhaltungen:
- EP-A2- 1 215 191
- US-B1- 6 580 005

## Beschreibung

Die vorliegende Erfindung betrifft ein Verfahren zur Gewinnung von aromatischen Dicarbonsäuren aus ihren jeweiligen Metallsalzen. Dieses Verfahren ist besonders geeignet, um in oder nach Verfahren eingesetzt zu werden, die eine basische Depolymerisation unter Zugabe von Metallhydroxiden aufweisen, mit dem Polykondensate wie beispielsweise PET aufgearbeitet werden, um die jeweiligen Monomere für einen erneuten Einsatz wiederzugewinnen.

Die in der Regel unmittelbar aus Basischemikalien fossilen Ursprunges hergestellten Polykondensate, insbesondere Polyethylenterephthalat-Kunststoffe (PET), aber auch Poly(ethylennaphthalate), Poly(ethylenterephthalat)-Poly(ethylenisophthalat)-Copolymere, Poly(butylenterephthalat)-Poly(ethyleneterephthalat)-Copolymere mit 1,4-Cyclohexandicarboxylat Struktureinheiten, werden in großem Umfang eingesetzt, u. anderem als Materialien für Getränkeflaschen, Lebensmittelverpackungen wie Salatschalen, Wurst- und Käseverpackungen, Fasern, Textilien, Automobilbauteile, klare, opake oder gefärbte Waschmittelflaschen. Es ist daher aus wirtschaftlichen wie Umweltschutzgründen wünschenswert, die Ausgangsmonomere dieser Polykondensate zurückzugewinnen, um sie einer erneuten Verwendung zuzuführen. Hierzu werden die aus diesen Polykondensaten hergestellten Produkte oder auch Produktionsabfälle recycelt, insbesondere mittels einer alkalischen Depolymerisation unter Zusatz von Alkoholen und Metallhydroxiden wie Natrium-, Kalium-, Magnesium-, Calcium-, und Bariumhydroxid. In diesen Recyclingverfahren erfolgen nach der alkalischen Depolymerisation die Schritte eines Lösens der in der Depolymerisation erzeugten Metallcarboxylate, einer Abtrennung nicht gelöster Bestandteile und gelöster Störstoffe, bevor die in der Depolymerisation erzeugten Metallcarboxylate mittels Säurezugabe ausgefällt werden. Anschließend kann das Fällungsprodukt als Rohprodukt zum Endprodukt aufgereinigt werden.

Die US 6 580 005 A beschreibt ein solches Recyclingverfahren zur Rückgewinnung von Terephthalsäure, bei dem zerkleinertes PET mit Polyethylenglykol und Natriumcarbonat versetzt wird, um Metallcarboxylat zu erhalten. Dieses wird in mehreren Schritten unter Einsatz von Schwefelsäure in die Terephthalsäure umgesetzt, wobei nach der Depolymerisation die Schritte einer Fest/flüssig-Trennung zur Abtrennung von Verunreinigungen einschließlich des Ethylenglykols, einer Neutralisation inklusive einer Kristallisation der Terephthalsäure erfolgen sowie deren Aufreinigung mittels Waschens, Fest/flüssig-Trennung und Trocknung.

Aus der US 6 031 128 A ist ein Verfahren zur Gewinnung einer reinen Terephthalsäure bekannt, bei dem ein ganz bestimmter, sortenreiner PET-Kunststoffabfall einem Recycling unterworfen wird, wobei im Anschluss an die basische Depolymerisation mittels NaOH eine Aufarbeitung der erhaltenen Aufschlämmung durch Fest/flüssig-Trennung und Weiterverarbeitung des in dem abgetrennten Lösemittel Wasser gelösten Metallcarboxylats erfolgt. Dazu wird letzteres nach Verdünnung mit maximal dem Dreifachen der benötigten Lösemittelmenge durch einen Aktivkohle-Adsorber geführt, um Verunreinigungen abzutrennen, wonach eine Neutralisation und Fällung mittels einer starken Säure wie HCl, H₂SO₄, HNO₃, H₃PO₄ erfolgt. Die ausgefallene feuchte Terephthalsäure wird anschließend in aufeinanderfolgenden Tanks bei jeweils fallender Temperatur wiederholt umkristallisiert, um größere und damit leichter abtrennbare Partikel zu erhalten. Abschließend erfolgt eine Kühlung unter reduziertem Druck, das Endprodukt wird nach Filtration und Trocknung erhalten.

Mit diesen Verfahren lassen sich jedoch nicht-sortenreine Abfälle nur schwer aufarbeiten. Die basische Depolymerisationsreaktion eines nichtsortenreinen Abfalls führt zu einem Ausgangsstoffgemisch für die weiteren Verfahrensschritte, das neben der Hauptkomponente der Metallcarboxylate in Form eines oder mehrerer Isomere eines oder unterschiedlicher Monomere des zu recycelnden Polykondensats, Reste des zugesetzten Metallhydroxids, Farbstoffe, Additive, Abbauprodukte und sonstige Verunreinigungen aus der Herstellung, Verarbeitung und Nutzung dieser Polykondensate enthält. Ebenso vorhanden sind organische Lösungsmittel und insbesondere die entsprechenden Monomeralkohole als Hydrolyseprodukte der Polykondensate und letztlich auch unvollständig umgesetzte Oligomere und Polymere der Polykondensate. Aufgrund der komplexen und variierenden Zusammensetzung dieses Ausgangsstoffgemisches ist dessen Aufarbeitung schwierig.

Die Erfindung stellt sich daher die Aufgabe, ein dementsprechend verbessertes Verfahren anzugeben.

Diese Aufgabe wird durch ein Verfahren gemäß Anspruch 1 gelöst, wobei vorteilhafte Weiterbildungen in den Unteransprüchen angegeben sind.

Ein erfindungsgemäßes Verfahren mit allen Verfahrensschritten läuft wie nachfolgend im Detail geschildert ab, wobei mindestens die Schritte des Lösens und Fällens sowie Abtrennens des Produktes zwingend vorhanden sein müssen.

In einem erfindungsgemäßen Vorbehandlungsschritt des aus der basischen Depolymerisationsreaktion stammenden Ausgangsstoffgemisches wird nur ein Teil der minimal zum vollständigen Lösen aller in diesem enthaltenen Metallcarboxylate benötigten Menge Lösungsmittel zugegeben, insbesondere nur diejenige Menge, die auf den Gehalt des am besten löslichen Isomers des Metallcarboxylats - oder der Metallcarboxylate bei Copolymeren - abgestimmt ist, so dass vor allem dieses in Lösung geht und somit vereinfacht aus dem Ausgangsstoffgemisch abtrennbar ist. Auf diese vorteilhafte Weise ist vor allem das oder die besser lösliche(n) Isomer(e) in höchster Isomerenreinheit zu einem sehr frühen Verfahrenszeitpunkt zur weiteren Aufarbeitung erhältlich, in dem die nur dieses Isomer enthaltende Lösung - insbesondere durch Filtration - vom restlichen Abstrom der Depolymerisation abgetrennt und das Filtrat aufgearbeitet wird._Die Abtrennung aus dem Abstrom der Depolymerisation erfolgt bevorzugt durch kontinuierliche als auch absatzweise arbeitende Filtrationsapparate, insbesondere Bandfilter, Taktbandfilter, Schrägbandfilter, Schmelzefilter oder Druckdrehfilter als auch Trennapparate, die die unterschiedliche Dichte der festen und flüssigen Suspensionsbestandteile ausnutzen. Sie führt zu einer flüssigen Phase, die neben dem Monomeralkohol - bei PET ist dies Ethylenglykol - wie geschildert nahezu ausschließlich das im Lösungsmittel besser lösliche Isomer des Metallcarboxylats, bzw. die besser löslichen Isomere, und das Lösungsmittel, insbesondere Wasser, enthält. In der weiteren Aufarbeitung dieses Filtrats wird der der Monomeralkohol vom Wasser insbesondere mittels Destillation, Rektifikation oder Membranverfahren, wie z. B. Pervaporation, abgetrennt. Das abgetrennte, besser lösliche Metallcarboxylat verbleibt ungelöst im Monomeralkohol und wird anschließend durch eine Fest/flüssig-Trennung aus diesem gewonnen. Alternativ kann sich auch der weiter unten beschriebene Schritt der reaktiven Fällung anschließen.

Auf diese Weise ist gleichzeitig auch das schlechter lösliche Isomer im Abstrom der Depolymerisation bereits aufgereinigt. Auf diese Weise sind mit Vorteil auch Verunreinigungen, die eine färbende Wirkung auf die spätere aromatische Dicarbonsäure des schlechter löslichen Isomers haben könnten, reduziert. Die Erfindung nimmt hierbei einen Verlust an dem oder den anderen, schwerer löslichen, Monomer(en) erfindungsgemäß in Kauf, sofern nur dieses, am besten lösliche, Isomer bzw. genauer dessen Dicarbonsäure erhalten werden soll. Ganz besonders vorteilhaft ist dieser, bezüglich des Gesamtgehalts an Metallcarboxylaten, nur für ein einziges Isomer erfolgende Lösungsschritt, da so die durch die eingangs genannten Copolymeren auftretenden weiteren Metallcarboxylaten schon zu diesem frühen Verfahrenszeitpunkt abgetrennt werden, so dass sie entweder gesondert aufgearbeitet oder entsorgt werden können. Erfindungsgemäß kann dieser erste Abtrennungsschritt bereits durch Zugabe einer entsprechenden Menge an Lösungsmittel in die Depolymerisationsreaktion selber erfolgen. Das Lösungsmittel ist dabei vorzugsweise Wasser oder eine wässrige Lösung eines Metallsulfats/-acetats wie Natriumsulfat oder Natriumacetat. Die wässrigen Natriumsulfat- und Acetatlösungen können dabei aus nachfolgenden Waschprozessen des erfindungsgemäßen Verfahrens zu diesem Schritt rückgeführt sein.

Alternativ zu diesem erfindungsgemäßen Vorbehandlungsschritt, der gezielt bevorzugt nur ein einziges Isomer abtrennt, wird dem Ausgangsstoffgemisch bei ansonst gleichem Vorgehen mindestens so viel Lösungsmittel zugegeben, dass das Metallcarboxylatisomerengemisch vollständig gelöst vorliegt. Hierbei werden zum Lösen des Metallcarboxylats Dinatriumterephthalat bei 20°C mindestens ein Liter Wasser pro 130 g Dinatriumterephthalat benötigt. Der Löseprozess wird apparativ vorteilhaft in einem Rührbehälter, einem statischen Mischer oder durch einen Rotor-Stator-Scherapparat (Inline-Dispergierer) durchgeführt In diesem alternativen Schritt ist es erfindungsgemäß auch möglich, dem Ausgangsstoffgemisch neben der Lösungsmittelzugabe- auch in Form einer wässrigen Sulfat/Acetat-Lösung - direkt eine definierte Menge derjenigen Säure zuzusetzen, die in einem nachfolgenden Verfahrensschritt der Fällungskristallisation in einem Fällungsmittel eingesetzt wird. Die zu diesem Verfahrenszeitpunkt einzusetzende Menge an Säure ist erfindungsgemäß dabei so gewählt, dass ein Metallhydroxidüberschuss neutralisiert wird, jedoch noch keine aromatische Dicarbonsäure auskristallisiert. Dies reduziert mit Vorteil erneut Verfärbungen, welche die Qualität des späteren Produkts vermindern. Es mindert ebenfalls mit Vorteil eine Korrosion von nachgeschalteten Anlagenkomponenten und bereitet das Ausgangsstoffgemisch in vorteilhafter Weise für eine nachfolgende Aufreinigung z.B. durch adsorptive Verfahren vor.

In einem dem Lösen nachfolgenden Schritt der Abtrennung nicht gelöster Störkomponenten wird die im letzten Schritt erhaltene Suspension von ungelösten Bestandteilen mittels Fest/flüssig-Trennung getrennt. Hierzu werden kontinuierliche und absatzweise Verfahren wie trägheitsbasierte Verfahren wie Schwimm-Sink-Separation sowie Filtrationsverfahren verwendet. Bei der Verwendung von Filtrationsverfahren werden diese vorzugsweise mehrstufig ausgeführt, typischerweise mit 2 - 8 Stufen und sinkender Trennkorngröße von bspw. 150 µm bis zu 0,5 µm absoluter Trennkorngröße. Die Filtration findet vorzugsweise bei Temperaturen unterhalb von 30°C statt, um auch Substanzen mit geringer Löslichkeit abzutrennen, die ansonsten in Lösung gehen würden. Ebenfalls erfindungsgemäß einsetzbar ist die Abtrennung feinster Feststoffpartikel durch Flokkulation mit Flockungsmitteln wie Polyaminen, Natriumaluminaten, Magnesiumchlorid, Eisensulfat und anderen zu größeren Agglomeraten, so dass die Fest/flüssig-Trennung einfacher und effizienter wird.

Die auf diese Weise erhaltene flüssige Phase wird im Anschluss einem Schritt der Abtrennung gelöster Störkomponenten wie löslichen Farbstoffen, UV-Stabilisatoren, Additiven, u.a.m. unterzogen, wobei erfindungsgemäß die gelösten Störkomponenten durch Adsorption an z. B. Aktivkohle, Zeolithe, Eisenhydroxidoxid oder durch Extraktionsverfahren abgetrennt werden. Als Aktivkohle wird jene mit geringer Affinität zu den gelösten Metallcarboxylaten gewählt, um Produktverluste zu vermeiden. Zudem erfolgt ggf. zur Verbesserung der Aufreinigung eine Anpassung des pH-Wertes durch Zugabe einer Säure, vorzugsweise ist dies die später als Fällungsmittel verwendete Säure. Werden Extraktionsverfahren eingesetzt, werden entweder die unerwünschten Störkomponenten oder die Metallcarboxylate in die Extraktphase überführt. Verwendbare Extraktionsmittel weisen entweder eine möglichst geringe Affinität zur Dicarbonsäure oder eine besonders hohe Affinität zur selektiven Gewinnung der Dicarbonsäure auf. Bevorzugt ist die Abtrennung der Störstoffe, um die Metallcarboxylate im wässrigen Medium zu belassen.

An den Schritt der Abtrennung gelöster Störkomponenten schließt sich erfindungsgemäß ein Schritt der reaktiven Fällung mittels Zugabe einer Säure an. Hierbei werden das oder die in der gereinigten Lösung befindlichen Metallcarboxylat(e) von der Säure aus ihren Salzen gedrängt und reagieren zur jeweiligen Dicarbonsäure, die aus der Lösung ausfällt.

Dieser besonders wichtige, erfindungsgemäße Schritt führt durch die Wahl einer Säure mit ganz bestimmten Eigenschaften zur Fällung einer aromatischen Dicarbonsäure mit optimierten Eigenschaften hinsichtlich Partikelgröße und Farbe, was deren Prozessierbarkeit hinsichtlich Filtrierbarkeit, Waschbarkeit, Schüttdichte und Fließeigenschaften der Mischung mit Monomeralkoholen deutlich verbessert. Zum anderen wird so eine besonders reine Dicarbonsäure erhalten, da die erfindungsgemäße Säure selektiv mit den Metallcarboxylaten der schlechter löslichen aromatischen Dicarbonsäure reagiert und die in der Lösung gegebenenfalls vorhandenen weiteren Metallcarboxylate der besser löslichen aromatischen Dicarbonsäuren durch die erfindungsgemäße Säure nur geringfügig ausgefällt werden. Mit anderen Worten werden am Beispiel von Gemischen der Benzoldicarbonsäuren die besser löslichen Isomere Phthalsäure und Isophthalsäure durch die erfindungsgemäße Säure deutlich geringfügiger ausgefällt und die schlechter lösliche Terephthalsäure jedoch in großem Maße.

Wird erfindungsgemäß im ersten Schritt der Vorbehandlung nur so wenig Lösemittel eingesetzt, dass vorrangig das am besten lösliche Isomer in der Lösung vorliegt und abgetrennt wurde, kann vorrangig auch nur dieses aus seiner Lösung ausfallen.

Neben der erfindungsgemäßen Verwendung einer Säure mit mindestens einem höherem pKs-Wert tragen auch die besonderen Prozessparameter der Fällung zur Gewinnung einer aromatischen Dicarbonsäure in hoher Reinheit und mit optimalen physikalischen Eigenschaften bei. Diese sind insbesondere eine Prozesstemperatur zwischen 50°C und 100°C, die überraschend größere aromatische Dicarbonsäurekristalle, insbesondere Terephthalsäurekristalle, entstehen lässt als eine höhere Temperatur, insbesondere bis zu 10fach größere Kristalle entstehen läßt. Größere Dicarbonsäurekristalle, insbesondere Terephthalsäurekristalle, erlauben eine effektivere und schnellere Fest/flüssig-Trennung. Gleichzeitig führt die Säurezugabe bei diesen Temperaturen und insbesondere bei 80°C - 95 C zu einer Reduktion der Kristallverfärbung. Schließlich führt diese Prozesstemperatur am Beispiel der Isomere der Terephthalsäure auch zu einem deutlich zu Terephthalsäure verschobenen Gleichgewicht im Kristallisat zwischen dieser und Isophthalsäure, sollte letztere noch in der Lösung verblieben sein. Das Verhältnis Terephthalsäure: Isophthalsäure liegt dabei mindestens bei 3:1.

Erfindungsgemäß wird dabei eine Säure eingesetzt, deren pKs-Wert größer als derjenige der schlechter löslichen aromatischen Dicarbonsäure, insbesondere von Terephthalsäure, ist. Es war daher für die Anmeldenden überraschend, dass eine solche schwache Säure für die Fällungsreaktion nicht nur überhaupt geeignet ist, sondern sogar besonders gut geeignet ist, da sie zu einem Terephthalsäurekristallisat von besonders vorteilhafter Größe für die weitere Prozessierung führt. Die erfindungsgemäß insbesondere für die Fällungsreaktion eingesetzte Ethansäure hat mit einem pKs-Wert von 4,76 eine geringere Säurestärke als Terephthalsäure mit einem pKs1-Wert von 3,54 und einem pKs2-Wert von 4,46. Die Verwendung und die Art der Zudosierung von Ethansäure in die Lösung von Metallcarboxylaten verschiedener Benzoldicarbonsäuren führt auch dazu, dass gezielt die weniger lösliche Säure, beispielsweise Terephthalsäure im Vergleich zu Isophthalsäure, aus ihrem Salz weitgehend selektiv gewonnen wird. Die im Stand der Technik üblicherweise verwendete Schwefelsäure führt demgegenüber bei äquimolarer Zugabe bezogen auf die Anzahl der verfügbaren und benötigten funktionellen Säuregruppen zu einer höheren Ausbeute an Terephthalsäure, ggf. allerdings im Gemisch mit Isophthalsäure. Mit anderen Worten nimmt die Erfindung eine schlechtere Gesamt-Isomeren-Ausbeute im Vergleich zur Verwendung von Schwefelsäure in Kauf, die eine Ausbeute von bis zu 99,9% ermöglicht, um ein reineres und besser verarbeitbares Produkt eines einzigen Dicarbonsäureisomers zu erhalten. Die Reduktion in der Ausbeute kann dabei bis zu 20% betragen.

Die Konzentration der Säure in dem Fällungsmittel liegt generell zwischen 1% und 100%. Die Fällung kann erfindungsgemäß auch bei einer Temperatur zwischen 100°C und 200°C und entsprechendem Dampfdruck erfolgen. Durch anschließende Kühlung der erhaltenen Suspension in geeigneter Weise werden Kristalle der aromatischen Dicarbonsäure gewonnen, die in ihrer Größe und Morphologie zum direkten Einsatz in herkömmlichen industriellen Prozessen für die Polymerisation geeignet sind, beispielsweise von Terephthalsäure zur Herstellung von PET .

In einem der Fällung nachfolgenden Schritt einer Fest/flüssig-Trennung und Wäsche des Fällungsprodukts und des Produkts der Rekristallisation wird die aus der reaktiven Fällung oder einer Rekristallisation stammende Suspension von Kristallen einer Fest/flüssig-Trennung unterzogen, um die aromatische Dicarbonsäure von ihrer Mutterlauge zu trennen. Hierzu werden kontinuierliche und absatzweise betriebene Apparate zur Fest/flüssig-Trennung eingesetzt, wie Bandfilter, Taktbandfilter, Drehtrommelfilter, Filterpressen, Filternutschen, Beutelfilter, Kerzenfilter, Siebfilter, Dekanterschneckenzentrifugen oder Tellerseparatoren. Die vor der Wäsche erhaltene Mutterlauge wird optional separat aufgefangen und teilweise oder vollständig als Lösungsmittel für das ursprüngliche Ausgangsstoffgemisch verwendet, wie eingangs erläutert. Hierbei ermöglicht die Verwendung von kontinuierlich arbeitenden Filtrationsapparaten wie Band-, Taktband- oder Drehtrommelfiltern, die direkte Wäsche des feuchten Filterkuchens mit einem Waschmedium, vorzugsweise Wasser.

Ein optionaler Schritt der Nachbehandlung in Form einer Rekristallisation der ausgefällten Dicarbonsäure oder der Mischung von Dicarbonsäuren kann sich erfindungsgemäß anschließen. In diesem werden die aus der reaktiven Fällung erhaltenen Kristalle durch Rekristallisation in Reinheit und morphologischen Eigenschaften optimiert. Das erneute Lösen der ausgefällten aromatischen Dicarbonsäure in einem Lösungsmittel mit anschließender Kristallisation führt zu einer Ausschleusung der im Kristall eingeschlossenen Verunreinigungen. Je nach temperaturabhängigem Löslichkeitsverhalten der Zieldicarbonsäure werden Kühlungs-, Verdampfungs- oder Flashkristallisationsverfahren angewendet. Die gesamte Rekristallisation erfolgt absatzweise oder kontinuierlich, in einer oder in 2 bis 6 Stufen, mit pro Stufe fallender Temperatur ausgehend von einer initialen maximalen Lösetemperatur, wobei eine kontinuierliche Rekristallisation bevorzugt ist. Bei Verwendung des Lösungsmittels Wassers für die Rekristallisation von Terephthalsäure sind Temperaturen von 180°C - 280°C bei entsprechenden Dampfdrücken zum Lösen vorteilhaft. Im Gegensatz zu herkömmlichen Aufreinigungs- und Kristallisationsverfahren industrieller Herstellungsprozesse hochreiner aromatischer Dicarbonsäuren ist es erfindungsgemäß mit großem Vorteil nicht notwendig, eine Temperatur und Konzentration zum vollständigen Lösen aller resuspendierten Kristalle zu wählen, da die aus dem Depolymerisationsprozess stammende aromatische Dicarbonsäure die typischen Verunreinigungen der herkömmlichen Herstellungsverfahren aromatischer Dicarbonsäuren nicht aufweist. Dies reduziert mit Vorteil Verfahrenskosten und -dauer. Die Ausschleusung von Fremdstoffen und die morphologische Anpassung sind dynamische Prozesse, welche auch auf Kristallreifungsprozessen basieren können. Dieser dynamische Löse- und Kristallisationsprozess kann je nach erforderlicher Reinheit zwischen 5 min und 300 min betragen. Fest/flüssig-Trennung und Wäsche des Kristallisats erfolgen analog zur oben beschriebenen Art und Weise.

Schließlich ist noch eine Trocknung des gewaschenen und mechanisch entfeuchteten Kristallisats vorgesehen. Dazu werden verschiedene absatzweise und kontinuierliche Trocknungsapparate eingesetzt wie Wirbelschichttrockner, Bandtrockner, Schaufelblatttrockner, Sprühtrockner oder Gefriertrockner. Die Trocknung erfolgt in 1 bis 4 Stufen.

### Ausführungsbeispiel 1

Eine pastöse Mischung aus 124,5 g Dinatriumterephthalat, 1,9 g Dinatriumisophthalat, 2,4 g Natriumhydroxid, 37,3 g Ethylenglykol, 2,1 g Polyethylenterephthalat Flakes und 3,9 g Polyethylen Flakes aus der alkalischen Estherhydrolyse eines PET/PE Multilayer Verpackungsmaterials wurde mit 1000 g vollentsalztem Wasser versetzt. Bei 20°C lösen sich die Salze der aromatischen Dicarbonsäuren vollständig und eine Suspension aus PE Flakes mit PET-Resten mit einem pH-Wert von 13,4 entsteht. Diese wurde mittels eines Spaltsiebs mit einer Spaltweite von 150 µm und anschließend mittels eines Siebfilters mit einer Maschenweite von 50 µm filtriert. Dabei konnten 95 Gew.-% der nicht gelösten Polymerbestandteile der Suspension abgetrennt werden. Durch eine anschließende Feinstfiltration mittels einer Polypropylenabsolutfilterkerze mit einer Filterfeinheit von 1 µm wurden 99,9 Gew.-% der verbleibenden nicht gelösten Bestandteile abgetrennt. Die erhaltene klare Lösung wurde unter Rühren durch Addition von 11 mL 25%iger Essigsäure auf einen pH-Wert von 9,8 eingestellt. Dabei wird nur das enthaltene Natriumhydroxid zu Natriumacetat neutralisiert. Zu dieser leicht gelblichen Lösung wurden anschließend 13,8 g Aktivkohlegranulat mit einer geringen Affinität zu den enthaltenen aromatischen Dicarbonsäuren gegeben, und die Suspension wurde unter Rühren auf 75 °C erwärmt. Nach der Verweilzeit von 50 min wurde die nun farblose Lösung mittels Filtration von der Aktivkohle befreit und in einen 1,5 L Glaskolben gegeben. Dort wurde die Lösung auf 80°C erwärmt und anschließend über eine Schlauchpumpe mit Dosierrohr mit 162,7 g 80%iger Essigsäure versetzt. Die eingesetzte Menge entspricht einem Stöchiometriefaktor von 3,6 bezogen auf die gesamte enthaltene Stoffmenge aromatischer Dicarbonsäuren und fällt 95 Gew.-% eben dieser aus. Die Spitze des Dosierrohrs befand sich apparativ direkt über den Blättern des verwendeten Propellerrührers, um eine optimale Durchmischung des Reaktionsmediums zu gewährleisten. Die Dosierdauer betrug 70 min und die nachfolgende Rührdauer 50 min bei 300 rpm. Anschließend wurde die Suspension auf 30 C abgekühlt, mittels einer Vakuumnutsche filtriert und zweistufig mit je 200 mL vollentsalztem Wasser gewaschen. Der Isophthalsäuregehalt des entstandenen Kristallisats betrug 0,2 Gew.-% (1,5 Gew.-% im Ausgangsstoff), ermittelt durch HPLC Messung mit UV/Vis Detektor.

30 g des feuchten Kristallisats mit einer Restfeuchte von 20 Gew.-% wurden mit 394 g vollentsalztem Wasser aufgeschlämmt und in einen gerührten 500 mL Druckreaktor gegeben. Bei 300 rpm wurde die Suspension auf 210°C erwärmt bei einem entsprechend resultierenden Dampfdruck von 18,5 bar. Die Verweilzeit bei 210°C betrug 60 min. Unter den genannten Prozessbedingungen lösen sich von den enthaltenen 24 g Kristallisat etwa 10 g. Im Anschluss wurde die Suspension stufenweise innerhalb von 90 min auf Umgebungstemperatur abgekühlt. Nach erneuter Filtration mittels Vakuumnutsche und anschließender Wäsche wurde das Kristallisat bei 85 C für 10 h getrocknet. Die Qualität des so erhaltenen Kristallisats kann nur mit dem zuvor beschriebenen Verfahren erreicht werden. Es entspricht den Anforderungen an Terephthalsäure zur Verwendung in industriellen Anlagen zur Herstellung von Polykondensaten. Hinsichtlich der Fließeigenschaften erreicht es in Suspensionen mit Ethylenglykol in molaren Verhältnissen von 1:1,1 bis 1:1,15 Viskositäten < 5 Pa*s bei 24°C. Bezüglich der Produktreinheit wird ein Farbwert von < 10°H erreicht.

### Ausführungsbeispiel 2

Eine pastöse Mischung aus 121,3 g Dinatriumterephthalat, 6,3 g Dinatriumisophthalat, 2,4 g Natriumhydroxid, 37,4 g Ethylenglykol, 2,1 g Polyethylenterephthalat-Flakes und 1,2 g Polyethylenterephthalat-Flakes aus der alkalischen Estherhydrolyse eines PET-Postconsumer-Verpackungsmaterials wurde mit 1.000 g vollentsalztem Wasser versetzt. Bei 20°C lösten sich die Salze der aromatischen Dicarbonsäuren vollständig und eine Suspension aus PET-Flakes mit einem pH-Wert von 13,4 entstand. Diese wurde mittels eines Filtertuchs mit einer Feinheit von 5-10 µm filtriert. Dabei wurden 98 Gew.-% der nicht gelösten Polymerbestandteile der Suspension abgetrennt. Durch eine anschließende Feinstfiltration mittels einer Polypropylenabsolutfilterkerze mit einer Filterfeinheit von 1 µm wurden 99,9 Gew.-% der verbliebenen nicht gelösten Bestandteile abgetrennt. Die Feinstklärung bis zu einer Feinheit von 50 nm erfolgte in einer nachfolgenden Polyethersulfon-Membranfilterkerze. Die erhaltene klare Lösung wurde unter Rühren durch Addition von 12,5 mL einer 25%igen Zitronensäure auf einen pH-Wert von 8,9 eingestellt. Dabei wurde nur das enthaltene Natriumhydroxid neutralisiert, wobei sich Natriumcitrat bildete. Zu dieser leicht gelblichen Lösung wurden anschließend 14 g Aktivkohlegranulat mit einer geringen Affinität zu den enthaltenen aromatischen Dicarbonsäuren gegeben und die Suspension unter Rühren auf 60 C erwärmt. Nach 60 min wurde die nun farblose Lösung mittels Filtration von der Aktivkohle befreit und in einen 1,5 L Glaskolben gegeben. Dort wurde die Lösung auf 65°C erwärmt und anschließend über eine Schlauchpumpe mit Dosierrohr mit 617 g einer 25%igen Zitronensäure versetzt entsprechend einem Stöchiometriefaktor von 1,33 bezogen auf die gesamte enthaltene Stoffmenge aromatischer Dicarbonsäuren. Hierdurch fielen 92 Gew.-% der enthaltenen aromatischen Dicarbonsäuren aus. Der Isophthalsäuregehalt des entstandenen Präzipitats betrug 2,8 Gew.-% (bei 5 Gew.-% im Ausgangsstoff), ermittelt durch HPLC Messung mit UV/Vis Detektor.

Alle weiteren Schritte folgten analog zu Ausführungsbeispiel 1.

### Ausführungsbeispiel 3

Die partikelfreie und farblose Lösung aus Ausführungsbeispiel 2 wurde auf 65 C erwärmt und in einem gerührten 1,5 L Glasreaktor mit 472,3 g 25 %iger ortho-Phosphorsäure versetzt, mithin bei einem Stöchiometriefaktor von 2. Hierbei fielen 87 % der enthaltenen aromatischen Dicarbonsäuren aus. Der Isophthalsäuregehalt des entstandenen Präzipitats betrug 2,7 Gew.-% (bei 5 Gew.-% im Ausgangsstoff, ermittelt durch HPLC Messung mit UV/Vis Detektor.

Das erfindungsgemäße Verfahren zur Gewinnung von aromatischen Dicarbonsäuren aus ihren Metallcarboxylaten unter Einsatz einer im Vergleich zu den Dicarbonsäuren schwächeren oder allenfalls gleichstarken Säure im Fällungsmittel führt überraschend zu einem hochreinen und in seinen physikalischen Eigenschaften für die spätere Verwendung in Polymerisationsreaktionen optimierten Produkt, wozu auch ein optionaler Schritt einer früh im Verfahrensablauf erfolgender Schritt der Abtrennung eines besser löslichen Metallcarboxylatisomers beiträgt.

## Patentansprüche

1. Verfahren zur Gewinnung von aromatischen Dicarbonsäuren aus einem Abstrom einer basischen Depolymerisationsreaktion von Polykondensaten enthaltend Metallcarboxylate der zu gewinnenden aromatischen Dicarbonsäure, bei dem diesem Abstrom eine mineralische oder organische Säure zugesetzt wird, die mindestens einen pKs-Wert größer oder gleich derjenigen der dem Polykondensat zugrundeliegenden aromatischen Dicarbonsäure aufweist.

2. Verfahren gemäß Anspruch 1, mit den Schritten a) Lösen der Metallcarboxylate in dem Abstrom, b) reaktive Fällung durch Zugabe der mineralischen und/oder organischen Säure und c) Abtrennung der ausgefällten Dicarbonsäure.

3. Verfahren gemäß Anspruch 1 oder 2, weiter aufweisend einen oder mehrere der Schritte: Abtrennung fester Störbestandteile des Abstroms, Abtrennung flüssiger und/oder gelöster Störbestandteile des Abstroms, Aufreinigung der ausgefällten Dicarbonsäure.

4. Verfahren gemäß Anspruch 3, bei dem der Schritt der Aufreinigung durch Rekristallisation bei Temperaturen zwischen 180°C und 280 C, bevorzugt zwischen 230°C und 275°C, derart erfolgt, dass nicht zwingend alle resuspendierten Kristalle gelöst werden.

5. Verfahren gemäß einem der vorherigen Ansprüche, bei dem Schritt a) mit einer solchen geringen Menge Lösungsmittel durchgeführt wird, dass nur das oder die besser lösliche(n) Metallcarboxylat(e) gelöst werden, wobei das Lösungsmittel bevorzugt Wasser ist.

6. Verfahren gemäß einem der vorherigen Ansprüche, bei dem die Säure des Fällungsmittels Ethansäure in reiner oder verdünnter Form ist.

7. Verfahren gemäß einem der vorherigen Ansprüche, bei dem die Säure direkt in die Lösung zugegeben wird, insbesondere über Rührblättern eines Rührers.

8. Verfahren gemäß einem der vorherigen Ansprüche, bei dem der Schritt der reaktiven Fällung bei einer Temperatur zwischen 50°C und 200°C, insbesondere zwischen 50°C und 100°C, durchgeführt wird.

9. Verfahren gemäß einem der vorherigen Ansprüche, bei dem alle Verfahrensschritte kontinuierlich durchgeführt werden.

## Claims

1. A method for extracting aromatic dicarboxylic acids from an effluent of a basic depolymerization reaction of polycondensates containing metal carboxylates of the aromatic dicarboxylic acid to be extracted, in which a mineral acid or an organic acid is added to this effluent and has at least a pKs value that is greater than or equal to that of the underlying aromatic dicarboxylic acid of the polycondensate.

2. The method according to claim 1, comprising the steps of a) dissolving the metal carboxylates in the effluent; b) carrying out a reactive precipitation by adding the mineral and/or organic acid; and c) separating the precipitated dicarboxylic acid.

3. The method according to claim 1 or 2, furthermore comprising one or more of the following steps: separating solid impurities of the effluent; separating liquid and/or dissolved impurities of the effluent; and purifying the precipitated dicarboxylic acid.

4. The method according to claim 3, in which the step of purifying by way of recrystallisation at temperatures between 180°C and 280°C, preferably between 230°C and 275°C, is carried out in such a way that not all resuspended crystals are necessarily dissolved.

5. The method according to any one of the preceding claims, in which step a) is carried out using such a small amount of solvent that only the better-soluble metal carboxylate(s) is/are dissolved, wherein the solvent is preferably water.

6. The method according to any one of the preceding claims, in which the acid of the precipitating agent is ethanoic acid in pure or diluted form.

7. The method according to any one of the preceding claims, in which the acid is added directly to the solution, in particular via agitator paddles of an agitator.

8. The method according to any one of the preceding claims, in which the step of reactive precipitation is carried out at a temperature between 50°C and 200°C, and in particular between 50°C and 100°C.

9. The method according to any one of the preceding claims, in which all method steps are carried out continuously.

## Revendications

1. Procédé d'extraction d'acides dicarboxyliques aromatiques depuis un effluent d'une réaction de dépolymérisation basique de polycondensats contenant des carboxylates métalliques de l'acide dicarboxylique aromatique à extraire, dans lequel un acide minéral ou un acide organique est ajouté à cet effluent et a au moins une valeur de pKs qui est supérieure ou égale à celle de l'acide dicarboxylique aromatique sous-jacent du polycondensat.

2. Procédé selon la revendication 1, comprenant les étapes consistant à a) dissoudre les carboxylates métalliques dans l'effluent ; b) mettre en œuvre une précipitation réactive en ajoutant l'acide minéral et/ou organique ; et c) séparer l'acide dicarboxylique précipité.

3. Procédé selon la revendication 1 ou 2, comprenant en outre une ou plusieurs des étapes suivantes consistant à :
séparer des impuretés solides de l'effluent ; séparer des impuretés liquides et/ou dissoutes de l'effluent ; et purifier l'acide dicarboxylique précipité.

4. Procédé selon la revendication 3, dans lequel l'étape consistant à purifier par recristallisation à des températures comprise entre 180 °C et 280 °C, préférentiellement entre 230 °C et 275 °C, est mise en œuvre d'une manière telle que tous les cristaux resuspendus sont nécessairement dissous.

5. Procédé selon l'une quelconque des revendications précédentes, dans lequel l'étape a) est mise en œuvre en utilisant une si petite quantité de solvant que seuls le ou les carboxylates métalliques les plus solubles sont dissous, dans lequel le solvant est préférentiellement l'eau.

6. Procédé selon l'une quelconque des revendications précédentes, dans lequel l'acide de l'agent de précipitation est de l'acide éthanoïque sous forme pure ou diluée.

7. Procédé selon l'une quelconque des revendications précédentes, dans lequel l'acide est ajouté directement à la solution, en particulier par l'intermédiaire des pales d'agitation d'un agitateur.

8. Procédé selon l'une quelconque des revendications précédentes, dans lequel l'étape de précipitation réactive est mise en œuvre à une température entre 50 °C et 200 °C, et en particulier entre 50 °C et 100 °C.

9. Procédé selon l'une quelconque des revendications précédentes, dans lequel toutes les étapes de procédé sont mises en œuvre de manière continue.
